# EUROPEAN PATENT APPLICATION

(11) **EP 1 547 579 A1**
(43) Date of publication of application: **29.06.2005**
(21) Application number: 03791233.4
(22) Date of filing: 20.08.2003
(51) Int. Cl.: A61K 9/00, A61K 9/70, A61K 47/10, A61K 47/30, A61K 47/32, A61K 47/34, A61K 47/36, A61K 47/38, A61K 47/42, A61K 47/46, A61K 31/573, A61P 5/44

(54) **ADHESIVE GEL COMPOSITION FOR IONTOPHORESIS PREPARATION AND PROCESS FOR PRODUCING THE SAME**

(30) Priority: 30.08.2002 JP 2002255682
(71) Applicant: HISAMITSU PHARMACEUTICAL CO., INC., Tosu-shi Saga 841-0017 (JP)
(72) Inventor: KURIBAYASHI, Mitsuru Tsukuba Laboratory, Tsukuba-shi, Ibaraki 305-0856 (JP); IKEDA, Shuichi Tsukuba Laboratory, Tsukuba-shi, Ibaraki 305-0856 (JP); TOKUMOTO, Seiji Tsukuba Laboratory, Tsukuba-shi, Ibaraki 305-0856 (JP); MORI, Kenji Tsukuba Laboratory, Tsukuba-shi, Ibaraki 305-0856 (JP); HIGO, Naruhito Tsukuba Laboratory, Tsukuba-shi, Ibaraki 305-0856 (JP)
(74) Representative: Westendorp, Michael, Dr.
(86) International application number: PCT/JP2003/010504
(87) International publication number: WO 2004/019902

(57) **Abstract**

The present invention provides an adhesive gel composition for iontophoretic formulations which is useful for the application through the skin or the mucosa, which excels in all of drug absorption, drug stability, gel shape retention and gel adhesion, which is easy to prepare and fill at the time of production, and which can be produced at low costs and a method for producing the same.

The adhesive gel composition for iontophoretic formulations is produced by mixing an ionic synthetic polymer(s), a nonionic synthetic polymer(s), a naturally-occurring polymer(s), a polyhydric alcohol(s), a crosslinking agent(s) and a drug(s). In the production of the adhesive gel composition for iontophoretic formulations, oxygen dissolved in the gel is positively removed by carrying out replacement with nitrogen and/or vacuum kneading at the time of the ingredients are mixed and kneaded.

## Description

### Technical Field

The present invention relates to an adhesive gel composition for iontophoretic formulations that is suitable for the application through the skin and the mucosa, and to a method for producing the same. In particular, the invention relates to an adhesive gel composition for iontophoretic formulations that includes ingredients such as ionic synthetic polymer, nonionic synthetic polymer, naturally-occurring polymer, polyhydric alcohol, crosslinking agent and drug, excels in shape retention and self-adhesion and that realizes high drug stability and drug absorption, and to a method for producing the same.

### Background Art

Iontophoresis is a method of delivering drugs through the skin or the mucosa by applying a voltage to the skin or the mucosa so as to electrically migrate ionic drugs. An iontophoresis device is used for medical treatment in such a manner as to place iontophoretic electrodes, for an anode and a cathode, on the skin at prescribed intervals and introduce a current generated by a current generator to the electrodes. The iontophoresis device is made up of the combination of a drug reserving layer and electrodes and includes not only a certain amount of active ingredient(s), which has(have) been designed to allow the blood level of the drug to be kept for a prescribed period of time, but various excipients required to keep stable efficacy of the drug. The drug reserving layer is divided into three major types: a "dropped drug impregnating type" layer into which a separately packaged drug solution is impregnated when applied; a "on-demand dissolving type" layer in which a drug is kept in the dried state and is dissolved when applied; and a "matrix type" layer in which a drug is mixed in advance in a hydrophilic gel base of, for example, a water-soluble polymer.

However, all the above pharmaceutical forms have problems of drug stability, handleability, drug absorption, etc., and to overcome such problems, there has been made a lot of invention until now.

For example, National-phase Publication of International Patent Application No. 8-503875 discloses an iontophoretic formulation of dropped drug impregnating type which is used by impregnating, at the time of medical treatment, a drug solution into its drug reserving layer made up of polyurethane foam, and a hydrophilic polymer in the dried state held in the polyurethane foam. With this formulation, the drug solution can be prevented from leaking out since the formulation has a hydrophilic polymer mixed therein, and at the same time, the adhesive between the drug reserving layer and the skin can be improved. However, the impregnation of the drug solution into the reserving layer takes a long time, and besides, the formulation is not satisfactory in operability and economy because of its complicated structure.

National-phase Publication of International Patent Application No. 7-507464 and Japanese Patent Laid-Open Nos. 11-192313 and 11-239621 disclose formulations of on-demand dissolving type in which a drug is held in the dried state in each drug holding layer and, when applied, it is dissolved and activated by hydrating the drug holding layer by hydrating means such as a water-containing gel or water- sealing capsule. These formulations are very suitable for drugs unstable in the presence of water; however, it is far from suitable for mass production because of the complexity of their structure, and besides, structurally there is possibility of water leaking from the hydrating means into the drug reserving layer, which poses a problem of quality assurance during storage.

In the meantime, there have also been developed hydrogel formulations which are produced by mixing a drug into a hydrophilic gel base of, for example, water-soluble polymer. As disclosed in Japanese Patent Laid-Open No. 63-92360 and Japanese Patent Publication No. 2-17187, gel compositions using naturally-occurring polymer such as agar and nonionic synthetic polymer such as polyvinyl alcohol have been known as hydrogel compositions for iontophoretic formulations of matrix type. However, these compositions have problems of poor adhesion and causing syneresis with the passage of time.

Thus, gel compositions of chemical crosslinking type using polyacrylic acid-based ionic synthetic polymer, which is often used in a poultice, have been examined, thinking of the solution of the above described problems.

For example, Japanese Patent Application No. 10-335602 discloses a composition, as a hydrogel composition for iontophoretic formulations of basic drugs, which is produced by mixing an acidic polymer such as polyacrylic acid or methoxyethylene-maleic anhydride copolymer, a polyhydric alcohol and gelatine and crosslinking the mixture with a polyfunctional epoxy compound. This gel composition has strong gel-shape retention and high adhesion. Further, it has the advantage of allowing pH setting without adding a special pH buffer, and therefore, making it unnecessary to fear the deterioration of drug absorption due to the competition of pH buffer etc. , since an acidic polymer itself has ionic functional groups.

However, it has been known that when an ionic drug or an electrolyte is mixed into a synthetic anionic polymer, the viscosity of the formulation is rapidly lowered, and the resultant formulation is in the liquid drossy state and is far from a gel body having sufficient shape retention. Thus, to obtain sufficient gel properties, it is necessary to decrease the amount of ionic drug mixed or increase the concentration of ionic synthetic polymer mixed. However, in either case, the problem occurs of decrease in drug release amount. Particularly for mixing of a high-concentration ionic synthetic polymer, it has been proved that such mixing causes decrease in transport number of a drug and increase in diffusion resistance of the same because the ionic synthetic polymer itself is conductive and it decreases the amount of free water, and therefore causing an extreme decrease in drug absorption in iontophoretic drug delivery. In this respect, an ionic synthetic polymer is a gel base for iontophoretic formulations which needs special attention because it has two conflicting effects in terms of gel properties and of drug absorption.

So then, in order to improve both gel properties and drug absorption, a base has been examined which is produced by introducing nonionic functional groups into a polyacrylic acid-based polymer. For example, Japanese Patent Laid-Open No. 5-97662 discloses a gel composition which uses acrylic acid and an acrylamide copolymer as a base and Japanese Patent Laid-Open No. 10-316590 discloses a gel composition using sodium acrylate and N-vinylacetoamide copolymer as a base. These bases have a decreased amount of anionic functional groups, and therefore, they can suppress their competition with drugs, which allows the minimization of decrease in drug absorption. These bases also suppress the decrease in gel viscosity caused by the mixing of ionic ingredients, thereby allowing the obtaining of stable gel properties.

However, in the above described bases, the amount of ionic functional groups as crosslinking points is too small to form a satisfactory gel body, and the formation of a satisfactory gel body requires a higher concentration of ionic ingredients, which is not economic in terms of their cost. The bases also have a problem of making it difficult to produce gel compositions when a high concentration of ionic functional groups are mixed thereinto, because the bases have thixotropic and non-Newtonian flow characteristics. Particularly in formulations prepared with the filling of gel in mind, it is hard to employ such compositions. Further, fluctuation in pH by hydrolysis etc. cannot be fully suppressed since the pH buffer capacity in gel is smaller compared with an ionic synthetic polymer. As a result, in compounds whose stability is affected by hydrolysis etc., it is very likely that sufficient stability cannot be ensured.

Specifically, conventional gel compositions for iontophoretic formulations have the following problems:
1) Gel compositions using a nonionic synthetic polymer or a naturally-occurring polymer as a base have poor adhesion and cause syneresis with the passage of time.
2) Gel compositions using an ionic synthetic polymer as a base cause abrupt decrease in the viscosity of formulations, resulting in decrease in the gel properties of the same. Accordingly, to obtain satisfactory gel properties, it is necessary to limit the amount of ionic ingredients mixed or mix an ionic synthetic polymer at a high concentration.
3) Ionic synthetic polymers are conductive polymers which decrease the amount of free water in gel, and therefore, when they are mixed at high concentrations, the drug absorption in iontophoretic drug delivery extremely deteriorate.
4) Gel compositions using, as a base, a weakly ionic synthetic polymer into which nonionic functional groups have been introduced can have gel properties that include high drug absorption. However, such a base has a problem in production of an intophoretic formulation. Specifically, to form a satisfactory gel body, it needs to be mixed at a high concentration since it does not have a sufficient amount of crosslinking points. Its thixotropic and non-Newtonian flow characteristics are also problematic in production of an intophoretic formulation.
5) Gel compositions using, as a base, a nonionic synthetic polymer and a weakly ionic synthetic polymer has a small pH buffer capacity since they have a small amount of dissociable functional groups. Accordingly, in compounds whose stability is affected by hydrolysis etc. , it is very likely that such gel compositions cannot ensure sufficient stability of the compounds.

As described so far, under the present circumstances, there have been known neither adhesive gel compositions for iontoporetic formulations which excels in all of drug absorption, drug stability, gel shape retention and gel adhesion; moreover, can be mass-produced nor a method for producing the same.

Accordingly, the present invention has been made so as to solve the above described problems, and its object is to provide an adhesive gel composition for iontophoretic formulations applied to drug delivery through the skin or the mucosa that excels in all of drug absorption, drug stability, gel shape retention and gel adhesion, is easy to prepare and fill at the time of production, and that can be produced at low costs and a method for producing the same.

### Disclosure of the Invention

After directing tremendous research efforts toward achieving the above described object, the present inventors found that such an object can be accomplished by an adhesive gel composition for iontophoretic formulations which is characterized by including an ionic synthetic polymer, a nonionic synthetic polymer, a naturally-occurring polymer, polyhydric alcohol, a crosslinking agent and a drug and by a method for producing the above adhesive gel composition for iontophoretic formulations which is characterized by positively removing oxygen dissolved in the gel by replacement with nitrogen and/or vacuum kneading. And they finally have completed the present invention.

Specifically, the present invention is an adhesive gel composition for iontophoretic formulations, characterized by including an ionic synthetic polymer(s), a nonionic synthetic polymer(s), a naturally-occurring polymer(s), a polyhydric alcohol (s), a crosslinking agent (s) and a drug(s).

The present invention is the above described adhesive gel composition for iontophoretic formulations, wherein the amount of the ionic synthetic polymer(s) mixed is 0.1 to 3.0% by weight, that of the nonionic synthetic polymer(s) is 0.5 to 30.0% byweight, that of the naturally-occurring polymer(s) is 0.5 to 10.0% by weight and that of the polyhydric alcohol(s) is 1.0 to 60.0% by weight.

The present invention is the above described adhesive gel composition for iontophoretic formulations, wherein the composition (weight) of the mixture of the ionic synthetic polymer(s) (A), the nonionic synthetic polymer(s) (B) and the naturally-occurring polymer(s) (C) is such that
(B + C)/A ≥ 1.5 % by weight and/or A + B + C ≥ 7% by weight.

The present invention is the above described adhesive gel composition for iontophoretic formulations, wherein the ionic synthetic polymer(s) is(are) obtained by polymerizing polymerizable unsaturated monomers that have at least anionic functional groups.

The present invention is the above described adhesive gel composition for iontophoretic formulations, wherein the ionic synthetic polymer(s) comprises one or more substances selected from the group consisting of polyacrylic acid, partially neutralized polyacrylic acid, fully neutralized polyacrylic acid, methoxyethylene-maleic anhydride copolymer, methoxyethylene-maleic acid copolymer, isobutylene-maleic anhydride copolymer, isobutylene-maleic acid copolymer and carboxyvinyl polymer.

The present invention is the above described adhesive gel composition for iontophoretic formulations, wherein the nonionic synthetic polymer(s) comprises one or more substances selected from the group consisting of polyvinyl alcohol, polyvinyl pyrrolidone and polyethylene oxide.

The present invention is the above described adhesive gel composition for iontophoretic formulations, wherein the naturally-occurring polymer(s) comprises one or more substances selected from the group consisting of gelatin, carrageenan, locust bean gum, dextrin, carboxymethyl cellulose and the metallic salt thereof.

The present invention is the above described adhesive gel composition for iontophoretic formulations, wherein the polyhydric alcohol(s) is one, or are two or more selected from the group consisting of glycerin, polyethylene glycol, propylene glycol, D-sorbitol, xylitol, mannitol and erythritol.

The present invention is the above described adhesive gel composition for iontophoretic formulations, wherein the crosslinking agent(s) is one, or are two or more selected from the group consisting of polyvalent metallic compounds, polyfunctional epoxy compounds and boric acid-based compounds.

The present invention is the above described adhesive gel composition for iontophoretic formulations, wherein the drug(s) form(s) anions in the adhesive gel composition for iontophoretic formulations and can be delivered from the cathode side of the iontophoretic formulations.

The present invention is the above described adhesive gel composition for iontophoretic formulations, wherein the drug(s) is(are) a water-soluble steroid hormone(s).

The present invention is the above described adhesive gel composition for iontophoretic formulations, wherein the water-soluble steroid hormone(s) is one, or are two or more selected from the group consisting of dexamethasone sodium phosphate, dexamethasone sodium acetate, dexamethasone sodium metasulfonbenzoate, hydrocortisone sodium succinate, hydrocortisone sodium phosphate, prednisolone sodium succinate and betamethasone sodium phosphate.

The present invention is the above described adhesive gel composition for iontophoretic formulations, wherein its pH is in the range of 4 to 9.

The present invention is the above described adhesive gel composition for iontophoretic formulations, wherein oxygen dissolved in the gel is positively removed by replacement with nitrogen and/or vacuum kneading at the time the ingredients are added and kneaded.

The present invention is a method for producing an adhesive gel composition for iontophoretic formulations, wherein oxygen dissolved in the gel is positively removed by carrying out replacement with nitrogen and/or vacuum kneading at the time of ingredients, including an ionic synthetic polymer(s), a nonionic synthetic polymer(s), a naturally-occurring polymer(s), a polyhydric alcohol(s), a crosslinking agent(s) and a drug(s), are mixed and kneaded.

### Brief Description of the Drawings

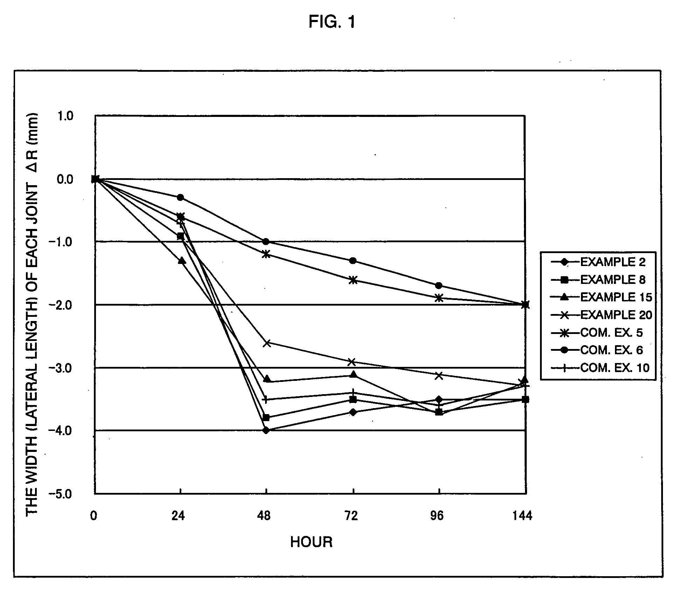

Figure 1 is a graph showing the results of a drug efficacy test for adhesive gel compositions for iontophoretic formulations in accordance with the present invention.

### Best Mode for Carrying Out the Invention

In.the following the present invention will be described in detail in terms of preferred embodiments.

The ionic synthetic polymers used in the adhesive gel composition for iontophoretic formulations of the present invention can be any synthetic polymers as long as they are hydrophilic bases having dissociable functional groups; however, preferably they are polymers obtained by polymerizing polymerizable unsaturated monomers having anionic functional groups. Preferred examples of such synthetic polymers include: polyacrylic acid, partially neutralized polyacrylic acid, fully neutralized polyacrylic acid, methoxyethylene-maleic anhydride copolymer, methoxyethylene-maleic acid copolymer, isobutylene-maleic anhydride copolymer, isobutylene-maleic acid copolymer, carboxyvinyl polymer, polyacrylamide, polyacrylamide derivatives, N-vinylacetoamide, and N-vinylacetoamide-acrylic acid and/or acrylate copolymer. Of these ionic synthetic polymers, polyacrylic acid, partially neutralized polyacrylic acid, fully neutralized polyacrylic acid, methoxyethylene-maleic anhydride copolymer, methoxyethylene-maleic acid copolymer, isobutylene-maleic anhydride copolymer, isobutylene-maleic acid copolymer and carboxyvinyl polymer are particularly preferable. These ionic synthetic polymers can be used individually or in the appropriate combination of two or more.

From the overall point of view, it is preferable that the above described dissociable functional groups include -COOH and -COOX (X: a counter ion) and preferably at least one carboxyl group.

These ionic synthetic polymers have the advantage of being capable of suppressing the competition with drugs, because they exist in state where charged functional groups are added to the polymer skeletons, and thus their transport number is very small compared with ordinary inorganic electrolytes. And the use of these ionic synthetic polymers is suitable for drugs which become less stable by hydrolysis etc., because the pH of the ionic synthetic polymers can be set, and at the same time, a great pH buffer capacity can be formed in them, depending on the combination. Further, the use of these ionic synthetic polymers contributes to preventing drug absorption from deteriorating, because it does not require positive addition of electrolytes at the time of pH adjustment.

The amount of the ionic synthetic polymers mixed is preferably 0.1 to 3.0% by weight of the total amount of the adhesive gel composition for iontophoretic formulations and more preferably 1.5 to 2.5% by weight. The amount outside the above described range is not preferable. Because if the amount is less than 0.1% by weight, neither shape retention nor pH buffer capacity of the gel composition can be formed satisfactorily, whereas if the amount is more than 3.0% by weight, the transport number of drugs is decreased and the diffusion resistance in the gel is increased by the ionic synthetic polymers mixed, resulting in extreme deterioration of the drug absorption of the gel.

The nonionic synthetic polymer used in the adhesive gel composition for iontophoretic formulations of the present invention can be any synthetic polymers as long as they are hydrophilic and nonionic. Preferred examples of such synthetic polymers include: polyvinyl alcohol, polyvinyl formal, polyvinyl methyl ether, polyvinyl methacrylate, polyvinyl pyrrolidone, polyvinyl pyrrolidone-vinyl acetate copolymer, polyethylene oxide and polypropylene oxide. Of these synthetic polymers, polyvinyl alcohol, polyvinyl pyrrolidone and polyethylene oxide are preferable. These nonionic synthetic polymers can be used individually or in the appropriate combination of two or more.

The amount of the nonionic synthetic polymers mixed is preferably 0.5 to 30.0% by weight of the total amount of the adhesive gel composition for iontophoretic formulations and more preferably 3.0 to 20.0% by weight. The amount outside the above described range is not preferable. Because if the amount is less than 0.5% by weight, neither shape retention nor adhesion of the gel composition can be produced satisfactorily, whereas if the amount is more than 30.0% by weight, the handleability and fillability of the gel composition in the production process deteriorate due to its thickening.

The naturally-occurring polymers used in the adhesive gel composition for iontophoretic formulations of the present invention can be either entirely naturally-occurring polymers or intentionally derivatized semi-synthetic polymers as long as they are compatible with the other ingredients. Examples of such naturally-occurring polymers include: gum arabic, gum tragacanth, locust bean gum, guar gum, echo gum, karraya gum, agar, starch, carrageenan, alginic acid and the metallic salt thereof, propylene glycol alginate, dextran, dextrin, amylose, gelatin, collagen, pullulan, pectin, amylopectin, starch, chitin, chitosan, albumin, casein, methyl cellulose, ethyl cellulose, propyl cellulose, ethyl methyl cellulose, hydroxymethyl cellulose, hydroxyethyl cellulose, hydroxypropyl methyl cellulose, carboxymethyl cellulose and the metallic salt thereof, hydroxypropyl starch, and starch-acrylic acid graft polymer. Of these naturally-occurring polymers, carrageenan, gelatin, locust bean gum, dextrin, carboxymethyl cellulose and the metallic salt thereof are preferable. These naturally-occurring polymers can be used individually or in the appropriate combination of two or more.

The amount of the naturally-occurring polymers mixed is preferably 0.5 to 10.0% by weight of the total amount of the adhesive gel composition for iontophoretic formulations and more preferably 2.0 to 6.5% by weight. The amount outside the above described range is not preferable. Because if the amount is less than 0.5% by weight, satisfactory gelling capacity cannot be produced due to the competition with the other ingredients, whereas if the amount is more than 10.0% by weight, the gel properties are good, but the handleability the gel composition in the production process deteriorate due to its thickening.

In the gel composition of this invention, the mixing ratio of the ionic synthetic polymer, the nonionic synthetic polymer and the naturally-occurring polymer is, within the range of the ionic synthetic polymer (A) 0.1 to 3.0% by weight, the nonionic synthetic polymer (B) 0.5 to 30.0% by weight and the naturally-occurring polymer (C) 0.5 to 30.0% byweight, preferably such that
(B + C)/A ≥ 1.5% by weight and/or A + B + C ≥ 7% by weight and more preferably such that
(B + C)/A ≥ 2.5 % by weight and/or A + B + C ≥ 9% by weight. If the above described mixing ratio is such that (B + C)/A < 1.5% by weight or A + B + C < 7% by weight , not only satisfactory gel shape retention cannot be produced, but also oozing occurs, and thus such mixing ratio is not preferable.

The polyhydric alcohols used in the adhesive gel composition for iontophoretic formulations of the present invention can be any polyhydric alcohols as long as they are compatible with the other ingredients. Preferred examples of such polyhydric alcohols include: glycols such as glycerin, ethylene glycol, diethylene glycol, triethylene glycol, polyethylene glycol, propylene glycol and polypropylene glycol; diols such as 1,3-propanediol and 1,4-butanediol; and sugar alcohols such as D-sorbitol, xylitol, mannitol and erythritol. Of these polyhydric alcohols, glycerin, polyethylene glycol, propylene glycol, D-sorbitol, xylitol, mannitol and erythritol are preferable and the combination of glycol and D-sorbitol is particularly preferable. These polyhydric alcohols can be used individually or in the appropriate combination of two or more. They function not only as plasticizers for the above described polymers, but also as fillers for promoting the moisture retention and the gel formation.

The amount of the polyhydric alcohols mixed is preferably 1.0 to 60.0% by weight of the total amount of the adhesive gel composition for iontophoretic formulations and more preferably 5.0 to 40.0% by weight. The amount outside the above described range is not preferable. Because if the amount is less than 1. 0% byweight, satisfactory cohesive power cannot be produced, and moreover, the water contained in the gel composition volatilizes, which might make it impossible to obtain satisfactory skin adhesion, whereas if the amount is more than 60.0% by weight, the gel strength becomes too high, causing deterioration of adhesion and oozing of liquid ingredients.

The crosslinking agents used in the adhesive gel composition for iontophoretic formulations of the present invention can be any crosslinking agents. Preferred examples of such crosslinking agents include: polyfunctional epoxy compounds, polyvalent metallic compounds and boric acid-based compounds. These crosslinking agents can be used individually or in the appropriate combination of two or more. Preferred examples of combinations of crosslinking agents are: the combination of polyvalent metallic compounds and boric acid-based compounds, in the gel compositions in which the amount of nonionic synthetic polymer and naturally-occurring polymer mixed is large; and the combination of polyfunctional epoxy compounds and polyvalent metallic compounds, in the gel compositions in which the amount of ionic synthetic polymer mixed is large.

Preferred examples of polyfunctional epoxy compounds include: sorbitol polyglycidyl ether, polyglycerin polyglycidyl ether, diglycerin polyglycidyl ether, glycerin polyglycidyl ether, ethylene glycol diglycidyl ether, polyethylene glycol diglycidyl ether and polypropylene glycol diglycidyl ether.

The amount of the polyfunctional epoxy compounds mixed, if they are used, is preferably 0.01 to 2.0% by weight of the total amount of the adhesive gel composition for iontophoretic formulations. The amount outside the above described range is not preferable. Because if the amount is less than 0.01% by weight, gelability cannot be obtained, whereas if the amount is more than 2% by weight, crosslinking progresses excessively and the adhesion of the gel deteriorates.

As polyvalent metallic compounds, aluminum compounds are preferable. Preferred examples of such aluminum compounds include: aluminum hydroxide gel, aluminum hydroxide, aluminum chloride, aluminum sulfate, magnesium metasilicate, dihydroxyaluminum aminoacetate, kaolin, aluminum stearate, magnesium aluminate metasilicate, synthetic hydotalsite, potassium aluminum sulfate, synthetic aluminum silicate, aluminum metasilicate, magnesium silicate and basic aluminum acetate.

The amount of the polyvalent metallic compounds mixed, if they are used, is preferably 0.01 to 5.0% by weight of the total amount of the adhesive gel composition for iontophoretic formulations and more preferably 0.3 to 3.0% by weight. The amount outside the above described range is not preferable. Because if the amount is less than 0.01% by weight, satisfactory gelling capacity cannot be produced due to their competition with the other ingredients, whereas if the amount is more than 5.0% by weight, the adsorption of drugs by crosslinking agents increases, which tends to cause the deterioration of the drug absorption of the gel composition.

Examples of boric acid-based compounds include: boric acid, ammonium borate, calcium borate, sodium metaborate and sodium tetraborate, which are suitably used as crosslinking agents for nonionic synthetic polymer or naturally-occurring polymer.

The amount of the boric acid-based compounds mixed, if they are used, is preferably 0.01 to 10% by weight of the total amount of the adhesive gel composition for iontophoretic formulations and more preferably 0.2 to 2.0% by weight. The amount outside the above described range is not preferable. Because if the amount is less than 0.01% by weight, a gel body having moldability cannot be obtained, whereas if the amount is more than 10% by weight, liquid ingredients, such as polyhydric alcohols and water, oozing from the gel surface due to excessive crosslinking.

The adhesive gel composition for iontophoretic formulations of this invention may contain a curing adjustor for adjusting the curing of metallic compounds. Examples of curing adjusters used include: organic acids such as lactic acid, tartaric acid and citric acid; and chelate compounds such as disodium edetate. The amount of the curing adjustor mixed, if it is used, is preferably, considering its competition with drugs, such that it does not cause the deterioration of the drug absorption of the gel at the time of iontophretic drug delivery.

In iontophretic drug delivery, water mixed in the adhesive gel composition for iontophoretic formulations of this invention is a very important ingredient for swelling the stratum corneum of the skin, lessening irritation to the skin, and dissolving and transmitting the drugs to be delivered.

The amount of water mixed is preferably 10 to 80% by weight of the total amount of the adhesive gel composition for iontophoretic formulations and more preferably 30 to 60% by weight. The amount outside the above described range is not preferable. Because if the amount is less than 10% by weight, the solubility of the water-soluble drug(s) in the composition deteriorates, causing the crystal deposition, or the amount of free water in the composition is decreased, causing the increase in diffusion resistance, which results in the decrease in drug absorption. On the other hand, if the amount is more than 80% by weight, a satisfactory gel body is hard to form, and moreover, the volatility of water is increased, which makes it possible to ensure the quality of the gel composition during storage and drug delivery.

The drugs contained in the adhesive gel composition of the present invention can be any drugs which are used in all the therapeutic areas, as long as they can dissolve in the adhesive gel composition and dissociate into anionic or cationic ions. Physiologically active substances with a molecular weight of 1 × 10² to 1 × 10⁶ can be particularly widely used.

Examples of drugs that can be used in the present invention include, not limited to, antiallergic, anesthetic, analgesic, antiasthmatic, anticonvulsant, antineoplastic, antipyretic, antiarrhythmic, hypotensive, diuretic, vasodilator, antiemetic, CNS stimulant, diagnostic, hormonic, anti-inflammatory, antidepressant, antipsychotic, immunosuppressant, muscle relaxant, antiviral, antibiotic, antithrombotic, bone absorption inhibitor and osteogenesis promoter. These drugs are used individually or in combination of two or more kinds.

Examples of drugs that can be dissociated into cations include, not limited to, bacampicillin, sultamicillin, cefpodoxime proxetil, cefteram pivoxil, cefmenoxime, cefotiam, doxycycline, minocycline, tetracyclin, erythromycin, rokitamycin, amikacin, arbekacin, astromicine, dibekacin, gentamicin, isepamicin, kanamycin, micronomicin, sisomicin, streptomycin, tobramycin, ethambutol, isoniazid, fluconazole, flucytosine, miconazole, aciclovir, chloramphenicol, clindamycin, fosfomycin, vancomycin, aclarubicin, bleomycin, cytarabine, dacarbazine, nimustine, peplomycin, procarbazine, vinblastine, vincristine, vindesine, calcitonins, parathyroid hormone (PTH), granulocyte-colony stimulating factor (G-CSF), mecasermin, alimemazine, chlorpheniramine, clemastine, mequitazine, azelastine, ketotifen, oxatomide, methylmethioninsulfonium chloride, colchicine, camostat, gabexate, nafamostat, mizoribine, piroxicam, proglumetacin, emorfazone, tiaramide, buprenorphine, ergotamine, phenacetin, rilmazafone, triazolam, zopiclone, nitrazepam, clonazepam, amantadine, bromocriptine, chlorpromazine, sultopride, chlordiazepoxide, cloxazolam, diazepam, etizolam, oxazolam, amitriptyline, imipramine, nortriptyline, setiptiline, ticlopidine, atropine, pancuronium bromide, tizanidine, pyridostigmine bromide, dobutamine, dopamine, benidipine, diltiazem, nicardipine, verapamil, acebutolol, atenolol, carteolol, metoprolol, nipradilol, pindolol, propranolol, dipyridamole, nicorandil, trapidil, ajimaline, aprindine, cibenzoline, disopyramide, flecainide, isoprenaline, lidocaine, mexoletine, procaine, procainamide, tetracaine, dibucaine, propafenone, quinidine, hydrochlorothiazide, trichlorothiazide, tripamide, azosemide, amosulalol, budralazine, bunazosin, cadralazine, clonidine, delapril, enalapril, guanethidine, hydralazine, labetalol, prazosin, reserpine, terazosin, urapidil, nicomol, epinephrine, etilefrine, midodrine, papaverine, clenbuterol, fenoterol, mabuterol, procaterol, salbutamol, terbutaline, tulobuterol, tipepidine, ambroxol, bromhexine, cimetidine, famotidine, ranitidine, roxatidine acetate, benexate, omeprazole, pirenzepine, sulpiride, cisapride, domperidone, metoclopramide, trimebutine, codeine, morphine, fentanyl, pethidine, oxybutynin, ritodrine, torodiline, and the salts thereof.

Examples of drugs that can be dissociated into anions include, not limited to, amoxicillin, ampicillin, aspoxicillin, benzylpenicillin, methicillin, piperacillin, sulbenicillin, ticarcillin, cefaclor, cefadroxil, cefalexin, cefatrizine, cefixime, cefradine, cefroxadine, cefamandole, cefazolin, cefmetazole, cefminox, cefoperazone, cefotaxime, cefotetan, cefoxitin, cefpiramide, cefsulodin, ceftazidime, ceftizoxime, ceftriaxone, cefuzonam, aztreonam, carumonam, flomoxef, imipenem, latamoxef, ciprofloxacin, enoxacin, nalidixic acid, norfloxacin, ofloxacin, vidarabine, fluorouracil, methotrexate, levothyroxine, liothyronine, amlexanox, cromoglicic acid, tranilast, gliclazide, insulins, prostaglandins, benzbromarone, carbazochrome, tranexamic acid, alclofenac, aspirin, diclofenac, ibuprofen, indometacin, ketoprofen, mefenamic acid, sulindac, tiaprofenic acid, tolmetin, sulpyrine, lobenzarit, penicillamine, amobarbital, pentobarbital, phenobarbital, thiopental, phenytoin, valproic acid, droxidopa, acetazolamide, bumetanide, canrenoic acid, etacrynic acid, alacepril, captopril, lisinopril, methyldopa, clofibrate, pravastatin, probucol, alprostadil, aminophylline, theophylline, carbocysteine, dexamethasone phosphate, dexamethasone acetate, dexamethasone metasulfonbenzoate, hydrocortisone succinate, hydrocortisone phosphate, prednisolone succinate, betamethasone phosphate and the salts thereof.

Of these drugs, those which form anions in the adhesive gel composition for iontophoretic formulations and can be delivered from the cathode side of the iontophoretic formulations are preferable. Preferred examples of such drugs are water-soluble steroid compounds, in particular, dexamethasone phosphate, dexamethasone acetate, dexamethasone metasulfonbenzoate, hydrocortisone succinate, hydrocortisone phosphate, prednisolone succinate, betamethasone phosphate and the salts thereof.

The reasons that drugs which form anions in the adhesive gel composition of iontophoretic formulations and can be delivered from the cathode side of the iontophoretic formulations are preferable are that: in the electrostatic interaction of anions with the ionic synthetic polymer in the gel composition, the interaction of the ionic synthetic polymer having anionic functional groups is smaller than that of the ionic synthetic polymer having cationic functional groups; and the drug delivery from the cathode side allows the gel to inhibit the movement (convective flow) of water from the skin side to the formulation side, which is a cause of deterioration of the drug absorption of the gel.

Use of the adhesive gel composition of the present invention makes possible the stable compounding of even drugs whose stability are very difficult to keep from hydrolysis and oxidative decomposition. The reasons for this are that the pH of the adhesive gel composition of the present invention can be set freely by appropriately combining the ingredients and that the adhesive gel composition of the present invention can form a strong pH buffer capacity, and thus it can suppress the pH fluctuation caused by hydrolysis etc., thereby decreasing the decomposition speed of drugs.

Further, according to the production method of the present invention, as described later, the ingredients of the gel composition can be compounded by nitrogen treatment or under vacuum, whereby the amount of oxygen dissolved in the gel can be greatly decreased, and hence the speed of drugs' decomposition byoxidation. Thus, drugs can also be used which cannot be mixed into a hydrophilic base by prior art.

The pH of the adhesive gel composition of the present invention is not particularly limited, since it varies depending on the stability of drugs or the type of the electrode from which drugs are delivered; however, taking into consideration the stability of drugs, the safety to the skin and the gel properties, the pH is preferably in the range of 4 to 9.

For example, when delivering dexamethasone sodium phosphate, it is preferable, from the viewpoint of suppressing hydrolysis and taking into consideration delivering anions from cathode side, to set the pH to weak alkali, that is, 7 to 9. When delivering the mixture of lidocaine and epinephrine, it is preferable, from the viewpoint of suppressing oxidative decomposition and taking into consideration delivering cations from anode side, to set the pH to acid, that is, 4 to 6. The pH can be adjusted by selecting the type of polymers as ingredients of the adhesive gel composition, and therefore, it is preferable not to positively add electrolytes, which lower the transport number of drugs.

The adhesive gel composition of the present invention may also contain additives such as stabilizer, preservative, absorption accelerator and pH adjustor, depending on the situation.

In the following, one example of methods for producing the adhesive gel composition of the present invention will be described; however, it is to be understood that the method is shown byway of example and not intended to limit the present invention.

In the production of the adhesive gel composition of the present invention, preferably replacement with nitrogen and/or vacuum kneading is carried out at the time ingredients are added and kneaded. Specifically, preferably ingredients are added and kneaded in such a state that the amount of oxygen dissolved in gel is decreased as much as possible by carrying out replacement with nitrogen and then vacuum operation using a vacuum kneader. And preferably the pre-treated ingredients are added to the kneader using a hopper so as to keep the vessel airtight. However, when it is inevitable to open the vessel, it is preferable to replace the atmosphere of the vessel with nitrogen and carry out vacuum operation again to suppress the oxygen concentration in the vessel. Preferably the degree of vacuum in the vessel during the preparation of the adhesive gel composition is appropriately set taking into consideration the expansion of the contents in the vessel or the efficiency of stirring, since it varies depending on the heating temperature. For example, when the heating temperature is 60°C or lower, the degree of vacuum can be set at - 0.08 MPa or less.

The procedure for preparing the adhesive gel composition for iontophoretic formulations of the present invention is not particularly limited; however, preferably the excipients are added from one having a strongest foaming tendency successively weaker so as not to make foams in the gel.

In one preferred example of preparing methods, naturally-occurring polymer formable at the time it is dissolved is previously dissolved in water by heating with a surfactant added thereto. And nonionic synthetic polymer is dispersed in a polyhydric alcohol. Both of these prepared materials are kneaded in a vacuum kneader while subjected to defoaming. Then, ionic synthetic polymer, a pre-treated preservative, that is, a preservative previously dispersed in a polyhydric alcohol, and drugs are added to the kneaded mixture one by one and dissolved uniformly. Lastly, a crosslinking agent, such as a polyvalent metallic compound or polyfunctional epoxy compound, dispersed or dissolved in a solvent is added, and the mixture is kneaded until it is brought to the uniform state. The adhesive gel composition of the present invention can be thus obtained.

The above described adhesive gel composition of the present invention is spread on an electrode, which has been formed in advance by printing the surface of a substrate with a conductive paste, to produce an electrode for iontophoresis, or it is spread on or filled into a film or a molding cup which has undergone release treatment in advance and then transferred and contact bonded to an electrode to produce an electrode for iontophoresis.

### (Examples)

In the following the present invention will be described in more detail giving examples and test examples; however, it is to be understood that these examples are not intended to limit the present invention. The term "part(s)" hereinafter used shall mean "part(s) by weight".

The mixing amounts of the ingredients used in examples 1 to 31 are shown in Tables 1 to 8 and those of the ingredients used in comparative examples 1 to 11 are shown in Tables 9 to 10.

### (Example 1)

7.5 parts of polyvinyl alcohol (fully saponified product: Kuraray Co., Ltd.) and 2.5 parts of carrageenan were added to 51.55 parts of water and dissolved by heating. Besides, 7.5 parts of polyvinyl pyrrolidone (K-90: ISP), 0.25 parts of polysodium acrylate (F-480ss: Showa Denko, K.K.), 0.18 parts of methyl parahydroxybenzoate and 0.02 parts of propyl parahydroxybenzoate were dispersed in 25 parts of glycerin. Both of the prepared materials were kneaded, while subjected to defoaming, using a vacuum kneader.

Then, 2.0 parts of D-sorbitol solution and 3.0 parts of dexamethasone sodium phosphate were added to the above kneaded material and dissolved uniformly. Lastly, 0.5 parts of sodium tetraborate was added and kneaded to give a uniform adhesive gel composition in accordance with the present invention.

In the production of the adhesive gel composition of the present invention, the addition of the prepared materials into the kneader was carried out with the kneading vessel opened, and once the vessel was opened, the atmosphere was replaced with nitrogen and vacuum operation was carried out.

0. 8 g of the resultant adhesive gel composition was filled into a polyester terephthalate convex molding vessel (24 mm in diameter, 1.5 mm in depth) which had been subjected to release treatment, and then a polyester terephthalate film was put on the adhesive gel composition with its release side facing the composition to give an adhesive gel composition of the present invention.

### (Examples 2 to 3)

Adhesive gel compositions of the present invention were obtained by the same method as used in example 1.

### (Example 4)

3.0 parts of gelatin, 5.0 parts of D-sorbitol solution and 0.3 parts of polyethylene glycol monostearate were added to 34.23 parts of water and dissolved by heating. Besides, 2 parts of polyvinyl alcohol (partially saponified product: Kuraray Co., Ltd.) and 2 parts of polyethylene oxide (coagulant: Dow Chemical Company) were dispersed in 17 parts of glycerin. Both of the prepared materials were kneaded, while subjected to defoaming, using a vacuum kneader.

Then, a material prepared by dispersing 1.0 part of polysodium acrylate (F-480ss: Showa Denko, K.K.), 0.5 parts of partially neutralized polyacrylic acid (NP-700: Showa Denko, K.K.), 0.18 parts of methyl parahydroxybenzoate and 0.02 parts of propyl parahydroxybenzoate in 10 parts of glycerin and a material prepared by dissolving 3 parts of dexamethasone sodium phosphate and 3.3 parts of β-cyclodextrin in 15 parts of water were added to the above kneaded material one by one and dissolved uniformly.

Lastly, a material prepared by dispersing 0.37 parts of dried aluminum hydroxide gel in 3 parts of glycerin and 0.1 parts of ethylene glycol diglycidyl ether were added and kneaded to give a uniform adhesive gel composition in accordance with the present invention.

In the production of the adhesive gel composition of the present invention, the addition of the prepared materials into the kneader was carried out with the kneading vessel opened, and once the vessel was opened, the atmosphere was replaced with nitrogen and vacuum operation was carried out.

0.8 g of the resultant adhesive gel composition was filled into a polyester terephthalate convex molding vessel (24 mm in diameter, 1.5 mm in depth) which had been subjected to release treatment, and then a polyester terephthalate film was put on the adhesive gel composition with its release side facing the composition to give an adhesive gel composition of the present invention.

### (Examples 5 to 25)

Adhesive gel compositions of the present invention were obtained by the same method as used in example 4.

### (Examples 26 to 28)

In example 26, an adhesive gel composition of the present invention was obtained by the same method as used in example 1 except that betamethasone sodium phosphate was used as a drug. In examples 27 to 28, adhesive gel compositions of the present invention were obtained by the same method as used in example 4 except that betamethasone sodium phosphate was used as a drug.

### (Examples 29 to 31)

In example 29, an adhesive gel composition of the present invention was obtained by the same method as used in example 1 except that lidocaine hydrochloride was used as a drug. In examples 30 to 31, adhesive gel compositions of the present invention were obtained by the same method as used in example 4 except that lidocaine hydrochloride was used as a drug.

### (Comparative examples 1 to 6)

Adhesive gel compositions of comparative examples 1 to 6 were obtained by the same method as used in example 4.

### (Comparative example 7)

12 parts of polyvinyl alcohol (fully saponified product: Unitika Ltd.) was added to 71 parts of water and dissolved by heating in an autoclave at 120°C for 15 minutes. Besides, 3 parts of dexamethasone sodium phosphate was dissolved in 14 parts of water. Both of the prepared materials were kneaded to give a uniform gel composition. 0.8 g of the resultant adhesive gel composition was filled into a polyester terephthalate convex molding vessel (24 mm in diameter, 1.5 mm in depth) which had been subjected to release treatment, and then a polyester terephthalate film was put on the adhesive gel composition with its release side facing the composition and frozen and crosslinked at - 80°C to give an adhesive gel composition.

### (Comparative example 8 to 9)

A gelling agent, methyl parahydroxybenzoate and propyl parahydroxybenzoate were added to a mixed solution of water and glycerin and dissolved by heating at around 60°C. Besides, dexamethasone sodium phosphate was dissolved in water. Both prepared materials were kneaded to give a uniform composition.

0.8 g of the resultant adhesive gel composition was filled into a polyester terephthalate convex molding vessel (24 mm in diameter, 1.5 mm in depth) which had been subjected to release treatment, and then a polyester terephthalate film was put on the adhesive gel composition with its release side facing the composition to give an adhesive gel composition.

### (Comparative example 10)

An adhesive gel composition was produced by the method of example 3 described in Japanese Patent Laid-Open No. 10-316590.

### (Comparative example 11)

An adhesive gel composition was produced by the same method as used in example 4 except that neither replacement with nitrogen nor vacuum treatment was carried out.

### (Test example 1: evaluation of gel properties)

Viscosity was measured for each of the adhesive gel compositions of examples 1 to 31 and comparative examples 1 to 11 right after their preparation. Then, each adhesive gel composition was left stand at room temperature for 14 days, and evaluation of gel properties and sensory test were carried out for each adhesive gel composition at the time when crosslinking was nearly completed. The test results for the compositions of examples 1 to 31 are shown in Table 1 to 8 and those for the compositions of comparative examples 1 to 11 are shown in Table 9 to 10.

The constitution ratios of the ionic synthetic polymer (A), nonionic synthetic polymer (B) and naturally-occurring polymer (C) mixed in the adhesive gel compositions are also shown in the respective tables.

### Evaluation of gel properties

### <pH>

The pH of the gel surface of each sample was measured using pH meter F-15 (HORIBA) with a plane glass electrode. Measurement was made 3 times for each sample, and the average of the three measurements was calculated as the pH of each sample.

### <Probe Tack>

Probe tack was measured for each sample using a probe tack tester equipped with a digital counter (Rigaku Denki Kogyo Co.) with a bakelite cylindrical (5 mm in diameter) probe. Measurement was made 3 times for each sample under the following conditions.
Pulling rate: 10 mm/sec
Contact time: 1 sec
Load 50 g (substantial load 30 g, ring load 20 g)

### <Viscosity>

Gel viscosity was measured for each sample right after its preparation using Visco tester VT-04 (RION). The temperature of the gel at the time of measurement was not standardized because the temperature for gel preparation differs depending on the ingredients; however, the measurement was made in the temperature range of 45°C to 60°C. Sensory Evaluation

Evaluation was made of skin adhesion, stickiness, gel shape retention, solvent- and water-oozing from gel and cup releasability. The evaluation criteria were as follows.

### <Skin adhesion>

Very good adhesion: ⓞ, Good adhesion: ○, Poor adhesion: Δ, No adhesion: ×.

### <Stickiness>

Does not leave the skin feeling sticky at all: ⓞ, Leaves the skin feeling sticky, but the stickiness dos not bother users: ○, Leaves the skin feeling rather sticky: Δ, Leaves the skin feeling very sticky: ×.

### <Gel shape retention>

Has a high gel strength or a high shape-memory and is not deformed: ⓞ,

Has a little low gel strength and is deformed, but has shape-memory: ○,

Has a low gel strength and partially fractures when deformed: Δ,

Deformed on very low impact and fully fractures: ×.

### <Oozing>

No oozing: ⓞ, Leaves the skin feeling slightly wet: ○, a small amount of oozing: Δ, a large amount of oozing: ×.

### <Cup releasability>

No gel left on the cup: ⓞ, A very small amount of gel left on the cup: ○, A small amount of gel left on the cup: Δ, The cup cannot be released or, when the cup is released, gel fractures: ×.

### <Overall evaluation>

Has very good properties and its use is not a problem at all: ⓞ, Has good properties and its use is not a problem: ○, Its use is a little problematic, but it is usable: Δ, Unusable: ×

**(Table 1)**

| (Examples 1 to 4) | | | | | |
|---|---|---|---|---|---|
| | Ingredients | 1 | 2 | 3 | 4 |
| Drug | dexamethasone sodium phosphate | 3 | 3 | 3 | 3 |
| | betamethasone sodium phosphate | | | | |
| | lidocaine hydrochloride | | | | |
| Ionic synthetic polymer (A) | polysodium acrylate | 0.25 | 0.5 | 0.5 | 1 |
| | partially neutralized polyacrylic acid | | | 0.5 | 0.5 |
| | polyacrylic acid | | | | |
| | carboxyvinyl polymer | | | | |
| | methoxyethylene-maleic anhydride copolymer | | | | |
| Nonionic synthetic polymer (B) | polyvinyl alcohol (partially saponified product) | | | | 2 |
| | polyvinyl alcohol (fully saponified product) | 7.5 | 7.5 | 7.5 | |
| | polyvinyl pyrrolidone polyethylene oxide | 7.5 | 7.5 | 7.5 | |
| | | | | | 2 |
| Naturally-oc curring polymer (C) | gelatin | | | | 3 |
| | carrageenan | 2.5 | 2.5 | 2.5 | |
| | cyclodextrin | | | | 3.3 |
| | locust bean gum | | | | |
| | carboxymethyl cellulose | | | | |
| Polyhydric alcohol | glycerin | 25 | 20 | 20 | 30 |
| | propylene glycol | | | | |
| | D-sorbitol solution (70%) | 2 | 2 | 2 | 5 |
| | erythritol | | | | |
| Crosslinking agent | dried aluminum hydroxide gel | | 0.37 | 0.37 | 0.37 |
| | ethylene glycol diglycidyl ether | | | | 0.1 |
| | urea | | | | |
| | synthetic aluminum silicate | | | | |
| | magnesium aluminate metasilicate | | | | |
| | sodium tetraborate | 0.5 | 0.5 | 0.5 | |
| pH adjuster | triethanolamine | | | | |
| Surfactant | polyethylene glycol monostearate | | | | 0.3 |
| Preservative | methyl paraben | 0.18 | 0.18 | 0.18 | 0.18 |
| | propyl paraben | 0.02 | 0.02 | 0.02 | 0.02 |
| | purified water | 51.55 | 55.93 | 55.43 | 49.23 |
| | A+B+C | 17.75 | 18 | 18.5 | 11.8 |
| | A/(A+B+C) | 1.41 | 2.78 | 5.41 | 12.71 |
| | (B+C)/A | 70.00 | 35.00 | 17.50 | 6.87 |
| Gel properties | pH | 6.62 | 6.96 | 6.61 | 7.62 |
| | Probe track (gF) | 13.3±1.0 | 14.5±3.4 | 13.0±2.4 | 7.8±0.5 |
| | viscosity (cps) | 15500 | 28000 | 32000 | 22500 |
| Sensory evaluation | skin adhesion | ○ | ⓞ | ⓞ | ○ |
| | stickiness | Δ | ○ | ⓞ | ○ |
| | shape retention | ⓞ | ⓞ | ⓞ | ⓞ |
| | oozing | ○ | ○ | ○ | ⓞ |
| | cup releasability | ⓞ | ○ | ⓞ | ⓞ |
| | overall evaluation (gel properties) | ○ | ○ | ○ | ⓞ |

**(Table 2)**

| (Examples 5 to 8) | | | | | |
|---|---|---|---|---|---|
| | Ingredients | 5 | 6 | 7 | 8 |
| Drug | dexamethasone sodium phosphate | 3 | 3 | 3 | 3 |
| | betamethasone sodium phosphate | | | | |
| | lidocaine hydrochloride | | | | |
| Ionic synthetic polymer (A) | polysodium acrylate | 2 | 2 | 1.5 | 1.5 |
| | partially neutralized polyacrylic acid | | | | 0.5 |
| | polyacrylic acid | | | | |
| | carboxyvinyl polymer | | | | |
| | methoxyethylene-maleic anhydride copolymer | | | 0.5 | |
| Nonionic synthetic polymer (B) | polyvinyl alcohol (partially saponified product) | 2 | 2 | 2 | 2 |
| | polyvinyl alcohol (fully saponified product) | | | | |
| | polyvinyl pyrrolidone | | | | |
| | polyethylene oxide | 2 | 1.5 | 1.5 | 2 |
| Naturally-oc curring polymer (C) | gelatin | 3 | 3.5 | 3.5 | 3 |
| | carrageenan | | | | |
| | cyclodextrin | 3.3 | 1.65 | 1.65 | |
| | locust bean gum | | | | |
| | carboxymethyl cellulose | | | | |
| Polyhydric alcohol | glycerin | 30 | 30 | 30 | 30 |
| | propylene glycol | | | | |
| | D-sorbitol solution (70%) | 5 | 3.75 | 3.75 | 5 |
| | erythritol | | | | |
| Crosslinking agent | dried aluminum hydroxide gel | 0.37 | 0.37 | 0.37 | 0.37 |
| | ethylene glycol diglycidyl ether | 0.1 | 0.1 | 0.1 | 0.1 |
| | urea | | | | |
| | synthetic aluminum silicate | | | | |
| | magnesium aluminate metasilicate | | | | |
| | sodium tetraborate | | | | |
| pH adjuster | triethanolamine | | | 1 | |
| Surfactant | polyethylene glycol monostearate | 0.3 | 0.3 | 0.3 | 0.3 |
| Preservative | methyl paraben | 0.18 | 0.18 | 0.18 | 0.18 |
| | propyl paraben | 0.02 | 0.02 | 0.02 | 0.02 |
| | purified water | 48.73 | 51.63 | 50.63 | 52.03 |
| | A+B+C | 12.3 | 10.65 | 10.65 | 9 |
| | A/(A+B+C) | 16.26 | 18.78 | 18.78 | 22.22 |
| | (B+C)/A | 5.15 | 4.33 | 4.33 | 3.5 |
| Gel properties | pH | 7.89 | 7.70 | 7.45 | 7.37 |
| | Probe track (gF) | 9.8±1.0 | 11.8±1.3 | 12.8±1.3 | 11.5±0.6 |
| | viscosity (cps) | 24000 | 24500 | 25000 | 23000 |
| Sensory evaluation | skin adhesion | ○ | ⓞ | ⓞ | ⓞ |
| | stickiness | ⓞ | ○ | ⓞ | ○ |
| | shape retention | ⓞ | ⓞ | ⓞ | ⓞ |
| | oozing | ⓞ | ⓞ | ⓞ | ⓞ |
| | cup releasability | ⓞ | ○ | ⓞ | ⓞ |
| | overall evaluation (gel properties) | ⓞ | ⓞ | ⓞ | ○ |

**(Table 3)**

| (Examples 9 to 12) | | | | | |
|---|---|---|---|---|---|
| | Ingredients | 9 | 10 | 11 | 12 |
| Drug | dexamethasone sodium phosphate | 3 | 3 | 3 | 3 |
| | betamethasone sodium phosphate | | | | |
| | lidocaine hydrochloride | | | | |
| Ionic synthetic polymer (A) | polysodium acrylate | 1 | | | 1.8 |
| | partially neutralized polyacrylic acid | 1 | 2 | 2 | 0.45 |
| | polyacrylic acid | | | | |
| | carboxyvinyl polymer | | | | |
| | methoxyethylene-maleic anhydride copolymer | | | | |
| Nonionic synthetic polymer (B) | polyvinyl alcohol (partially saponified product) | 2 | 2 | 1 | 2 |
| | polyvinyl alcohol (fully saponified product) | | | | |
| | polyvinyl pyrrolidone | | | | |
| | polyethylene oxide | 1 | | | 1.5 |
| Naturally-oc curring polymer (C) | gelatin | 3 | 3 | 3 | |
| | carrageenan | | | | 2.5 |
| | cyclodextrin | | | | |
| | locust bean gum | | | | |
| | carboxymethyl cellulose | | | | |
| Polyhydric alcohol | glycerin | 30 | 30 | 30 | 30 |
| | propylene glycol | | | | |
| | D-sorbitol solution (70%) | 5 | 2.5 | 3 | 3 |
| | erythritol | | | | |
| Crosslinking agent | dried aluminum hydroxide gel | 0.37 | 0.37 | 0.37 | 0.37 |
| | ethylene glycol diglycidyl ether | 0.1 | 0.1 | 0.1 | 0.1 |
| | urea | 2.5 | | | |
| | synthetic aluminum silicate | | | | |
| | magnesium aluminate metasilicate | | | | |
| | sodium tetraborate | | | | |
| pH adjuster | triethanolamine | 0.5 | | 0.5 | 1 |
| Surfactant | polyethylene glycol monostearate | 0.3 | 0.3 | 0.5 | 0.3 |
| Preservative | methyl paraben | 0.18 | 0.18 | 0.18 | 0.18 |
| | propyl paraben | 0.02 | 0.02 | 0.02 | 0.02 |
| | purified water | 50.03 | 56.53 | 56.53 | 53.78 |
| | A+B+C | 8 | 7 | 6 | 8.25 |
| | A/(A+B+C) | 25.00 | 28.57 | 33.33 | 27.27 |
| | (B+C)/A | 3.00 | 2.50 | 2.00 | 2.67 |
| Gel properties | pH | 7.00 | 6.42 | 7.15 | 8.07 |
| | Probe track (gF) | 8.3±1.3 | 10.5±0.6 | 10.5±0.6 | 11.3±0.5 |
| | viscosity (cps) | 22500 | 20000 | 15000 | 35500 |
| Sensory evaluation | skin adhesion | ○ | ⓞ | ⓞ | ⓞ |
| | stickiness | Δ | Δ | Δ | ○ |
| | shape retention | ⓞ | Δ | Δ | ○ |
| | oozing | ○ | ⓞ | ⓞ | ⓞ |
| | cup releasability | ○ | ○ | ○ | ○ |
| | overall evaluation (gel properties) | ○ | Δ | Δ | ○ |

**(Table 4)**

| (Examples 13 to 16) | | | | | |
|---|---|---|---|---|---|
| | Ingredients | 13 | 14 | 15 | 16 |
| Drug | dexamethasone sodium phosphate | 3 | 3 | 3 | 3 |
| | betamethasone sodium phosphate | | | | |
| | lidocaine hydrochloride | | | | |
| Ionic synthetic polymer (A) | polysodium acrylate | 2.5 | 2 | 1.5 | 2.5 |
| | partially neutralized polyacrylic acid | | 0.4 | | |
| | polyacrylic acid | | | 1 | |
| | carboxyvinyl polymer | | 0.1 | | |
| | methoxyethylene-maleic anhydride copolymer | | | | |
| Nonionic synthetic polymer (B) | polyvinyl alcohol (partially saponified product) | 2 | 2 | 2 | 2 |
| | polyvinyl alcohol (fully saponified product) | | | | |
| | polyvinyl pyrrolidone | | | | |
| | polyethylene oxide | 1.5 | 1.5 | | 1.5 |
| Naturally-oc curring polymer (C) | gelatin | 3.5 | 3 | 3 | |
| | carrageenan | | | | 2 |
| | cyclodextrin | 1.65 | | | |
| | locust bean gum | | | | |
| | carboxymethyl cellulose | | | | |
| Polyhydric alcohol | glycerin | 30 | 30 | 30 | 22.5 |
| | propylene glycol | | | | |
| | D-sorbitol solution (70%) | 3 | 4.9 | 5 | 3 |
| | erythritol | | | | |
| Crosslinking agent | dried aluminum hydroxide gel | 0.37 | 0.37 | 0.37 | 0.37 |
| | ethylene glycol diglycidyl ether | 0.1 | 0.1 | 0.1 | 0.1 |
| | urea | | | | |
| | synthetic aluminum silicate | | | | |
| | magnesium aluminate metasilicate | | | | |
| | sodium tetraborate | | | | |
| pH adjuster | triethanolamine | | 0.5 | 2 | |
| Surfactant | polyethylene glycol monostearate | 0.3 | 0.3 | 0.5 | 0.3 |
| Preservative | methyl paraben | 0.18 | 0.18 | 0.18 | 0.18 |
| | propyl paraben | 0.02 | 0.02 | 0.02 | 0.02 |
| | purified water | 51.88 | 51.63 | 51.33 | 62.53 |
| | A+B+C | 11.15 | 9 | 7.5 | 8 |
| | A/(A+B+C) | 22.42 | 27.78 | 33.33 | 31.25 |
| | (B+C)/A | 3.46 | 2.60 | 2.00 | 2.20 |
| Gel properties | pH | 7.71 | 7.50 | 7.37 | 7.58 |
| | Probe track (gF) | 12.8±1.0 | 11.8±0.5 | 14.0±0.5 | 10.3±1.0 |
| | viscosity (cps) | 27000 | 28500 | 23000 | 37000 |
| Sensory evaluation | skin adhesion | ⓞ | ⓞ | ⓞ | ○ |
| | stickiness - | ○ | ⓞ | ⓞ | ⓞ |
| | shape retention | ⓞ | ⓞ | ○ | ○ |
| | oozing | ⓞ | ⓞ | ⓞ | ⓞ |
| | cup releasability | ⓞ | ⓞ | ⓞ | ○ |
| | overall evaluation (gel properties) | ⓞ | ⓞ | ⓞ | ○ |

**(Table 5)**

| (Examples 17 to 20) | | | | | |
|---|---|---|---|---|---|
| | Ingredients | 17 | 18 | 19 | 20 |
| Drug | dexamethasone sodium phosphate | 3 | 3 | 3 | 3 |
| | betamethasone sodium phosphate | | | | |
| | lidocaine hydrochloride | | | | |
| Ionic synthetic polymer (A) | polysodium acrylate | 2 | 2 | 2 | 2 |
| | partially neutralized polyacrylic acid | 0.5 | 0.5 | 0.5 | 1 |
| | polyacrylic acid | | | | |
| | carboxyvinyl polymer | | | | |
| | methoxyethylene-maleic anhydride copolymer | | | | |
| Nonionic synthetic polymer (B) | polyvinyl alcohol (partially saponified product) | 2 | 2 | 2 | 2.5 |
| | polyvinyl alcohol (fully saponified product) | | | | |
| | polyvinyl pyrrolidone | | | | |
| | polyethylene oxide | 1.5 | 1.5 | 1.5 | 0.5 |
| Naturally-oc curring polymer (C) | gelatin | | | | 3 |
| | carrageenan | 2 | 3 | 1.5 | |
| | cyclodextrin | | | | |
| | locust bean gum | | | 0.75 | |
| | carboxymethyl cellulose | 1 | | | |
| Polyhydric alcohol | glycerin | 30 | 30 | 30 | 30 |
| | propylene glycol | | | | |
| | D-sorbitol solution (70%) | 5 | | 3 | 3 |
| | erythritol | | 5 | | |
| Crosslinking agent | dried aluminum hydroxide gel | 0.37 | 0.37 | 0.37 | 0.37 |
| | ethylene glycol diglycidyl ether | 0.1 | 0.1 | 0.1 | 0.1 |
| | urea | | | | |
| | synthetic aluminum silicate | | | | |
| | magnesium aluminate metasilicate | | | | |
| | sodium tetraborate | | | | |
| pH adjuster | triethanolamine | | | | 0.5 |
| Surfactant | polyethylene glycol monostearate | 0.3 | 0.3 | 0.5 | 0.3 |
| Preservative | methyl paraben | 0.18 | 0.18 | 0.18 | 0.18 |
| | propyl paraben | 0.02 | 0.02 | 0.02 | 0.02 |
| | purified water | 52.03 | 52.03 | 54.78 | 53.53 |
| | A+B+C | 9 | 9 | 8.25 | 9 |
| | A/(A+B+C) | 27.78 | 27.78 | 30.30 | 33.33 |
| | (B+C)/A | 2.60 | 2.60 | 2.30 | 2.00 |
| Gel properties | pH | 7.48 | 7.68 | 7.75 | 7.80 |
| | Probe track (gF) | 12.45±0. 6 | 12.0±0.0 | 8.0±0.8 | 8.8±1.2 |
| | viscosity (cps) | 49000 | 41000 | 45000 | 30000 |
| Sensory evaluation | skin adhesion | ⓞ | ⓞ | ○ | ○ |
| | stickiness | ○ | ○ | ○ | ⓞ |
| | shape retention | Δ | Δ | ○ | ⓞ |
| | oozing | ⓞ | ⓞ | ⓞ | ⓞ |
| | cup releasability | ⓞ | ⓞ | ⓞ | ⓞ |
| | overall evaluation (gel properties) | ○ | ○ | ○ | ⓞ |

**(Table 6)**

| (Examples 21 to 24) | | | | | |
|---|---|---|---|---|---|
| | Ingredients | 21 | 22 | 23 | 24 |
| Drug | dexamethasone sodium phosphate | 3 | 3 | 3 | 3 |
| | betamethasone sodium phosphate | | | | |
| | lidocaine hydrochloride | | | | |
| Ionic synthetic polymer (A) | polysodium acrylate | 2 | 2 | 2 | 2 |
| | partially neutralized polyacrylic acid | 1 | 1 | 1 | |
| | polyacrylic acid | | | | |
| | carboxyvinyl polymer | | | | |
| | methoxyethylene-maleic anhydride copolymer | | | | |
| Nonionic synthetic polymer (B) | polyvinyl alcohol (partially saponified product) | 2 | 2 | 1.5 | 3 |
| | polyvinyl alcohol (fully saponified product) | | | | |
| | polyvinyl pyrrolidone | | | | |
| | polyethylene oxide | 0.5 | | | |
| Naturally-oc curring polymer (C) | gelatin | 2.5 | 2.5 | 2.5 | 3 |
| | carrageenan | | | | |
| | cyclodextrin | | | | |
| | locust bean gum | | | | |
| | carboxymethyl cellulose | | | | |
| Polyhydric alcohol | glycerin | 30 | 30 | 30 | 30 |
| | propylene glycol | | | | |
| | D-sorbitol solution (70%) | 3 | 3 | | |
| | erythritol | | | | |
| Crosslinking agent | dried aluminum hydroxide gel | 0.37 | 0.37 | | 0.37 |
| | ethylene glycol diglycidyl ether | 0.1 | 0.1 | 0.1 | |
| | urea | | | | 5 |
| | synthetic aluminum silicate | | | | 1 |
| | magnesium aluminate metasilicate | | | | |
| | sodium tetraborate | | | | |
| pH adjuster | triethanolamine | 0.5 | 0.5 | 0.5 | |
| Surfactant | polyethylene glycol monostearate | 0.3 | 0.3 | 0.3 | |
| Preservative | methyl paraben | 0.18 | 0.18 | 0.18 | 0.18 |
| | propyl paraben | 0.02 | 0.02 | 0.02 | 0.02 |
| | purified water | 54.53 | 55.03 | 58.9 | 51.43 |
| | A+B+C | 8 | 7.5 | 7 | 9 |
| | A/(A+B+C) | 37.50 | 40.00 | 42.86 | 33.33 |
| | (B+C)/A | 1.67 | 1.50 | 1.33 | 2.00 |
| Gel properties | pH | 7.83 | 6.07 | 6.27 | 6.07 |
| | Probe track (gF) | 10.8±1.5 | 7.3±1.0 | 8.2±1.0 | 7.3±1.0 |
| | viscosity (cps) | 27500 | 26500 | 25000 | 26500 |
| Sensory evaluation | skin adhesion | ⓞ | ○ | Δ | ⓞ |
| | stickiness | ⓞ | ⓞ | Δ | ○ |
| | shape retention | ⓞ | ○ | Δ | ○ |
| | oozing | ⓞ | ⓞ | ⓞ | ○ |
| | cup releasability | ⓞ | ⓞ | ⓞ | ⓞ |
| | overall evaluation (gel properties) | ⓞ | ○ | Δ | ○ |

**(Table 7)**

| (Examples 25 to 28) | | | | | |
|---|---|---|---|---|---|
| | Ingredients | 25 | 26 | 27 | 28 |
| Drug | dexamethasone sodium phosphate | 3 | | | |
| | betamethasone sodium phosphate | | 3 | 3 | 3 |
| | lidocaine hydrochloride | | | | |
| Ionic synthetic polymer (A) | polysodium acrylate | 2 | 0.5 | 2 | 2 |
| | partially neutralized polyacrylic acid | | | | 0.5 |
| | polyacrylic acid | 1 | | | |
| | carboxyvinyl polymer | | | | |
| | methoxyethylene-maleic anhydride copolymer | | | | |
| Nonionic synthetic polymer (B) | polyvinyl alcohol (partially saponified product) | 3 | | 2 | 2 |
| | polyvinyl alcohol (fully saponified product) | | 7.5 | | |
| | polyvinyl pyrrolidone | | 7.5 | | |
| | polyethylene oxide | | | 1.5 | 1.5 |
| Naturally-oc curring polymer (C) | gelatin | 3 | | 3.5 | |
| | carrageenan | | 2.5 | | 3 |
| | cyclodextrin | | | 1.65 | |
| | locust bean gum | | | | |
| | carboxymethyl cellulose | | | | |
| Polyhydric alcohol | glycerin | 30 | 20 | 30 | 30 |
| | propylene glycol | | | | |
| | D-sorbitol solution (70%) | | 2 | 3.75 | 5 |
| | erythritol | | | | |
| Crosslinking agent | dried aluminum hydroxide gel | 0.37 | 0.37 | 0.37 | 0.37 |
| | ethylene glycol diglycidyl ether | | | 0.1 | 0.1 |
| | urea | 5 | | | |
| | synthetic aluminum silicate | | | | |
| | magnesium aluminate metasilicate | 1 | | | |
| | sodium tetraborate | | 0.5 | | |
| pH adjuster | triethanolamine | | | | |
| Surfactant | polyethylene glycol monostearate | | | 0.3 | 0.3 |
| Preservative | methyl paraben | 0.18 | 0.18 | 0.18 | 0.18 |
| | propyl paraben | 0.02 | 0.02 | 0.02 | 0.02 |
| | purified water | 51.43 | 55.93 | 51.63 | 52.03 |
| | A+B+C | 9 | 18 | 10.65 | 9 |
| | A/(A+B+C) | 33.33 | 2.78 | 18.78 | 27.78 |
| | (B+C)/A | 2.00 | 35.00 | 4.33 | 2.60 |
| Gel properties | pH | 6.27 | 6.56 | 7.70 | 7.58 |
| | Probe track (gF) | 8.2±1.0 | 15.3±1.9 | 11.8±1.3 | 12.0±0.0 |
| | viscosity (cps) | 25000 | 22500 | 24500 | 40000 |
| Sensory evaluation | skin adhesion | ⓞ | ○ | ⓞ | ⓞ |
| | stickiness | ○ | ○ | ○ | ○ |
| | shape retention | ⓞ | ⓞ | ⓞ | Δ |
| | oozing | ○ | ○ | ⓞ | ⓞ |
| | cup releasability | ⓞ | ⓞ | ○ | ⓞ |
| | overall evaluation (gel properties) | ○ | ○ | ○ | ○ |

**(Table 8)**

| (Examples 29 to 31) | | | | |
|---|---|---|---|---|
| | Ingredients | 29 | 30 | 31 |
| Drug | dexamethasone sodium phosphate | | | |
| | betamethasone sodium phosphate | | | |
| | lidocaine hydrochloride | 3 | 3 | 3 |
| Ionic synthetic polymer (A) | polysodium acrylate | 0.5 | 2 | 2 |
| | partially neutralized polyacrylic acid | | | 0.5 |
| | polyacrylic acid | | | |
| | carboxyvinyl polymer | | | |
| | methoxyethylene-maleic anhydride copolymer | | | |
| Nonionic synthetic polymer (B) | polyvinyl alcohol (partially saponified product) | | 2 | 2 |
| | polyvinyl alcohol (fully saponified product) | 7.5 | | |
| | polyvinyl pyrrolidone | 7.5 | | |
| | polyethylene oxide | | 1.5 | 1.5 |
| Naturally-oc curring polymer (C) | gelatin | | 3.5 | |
| | carrageenan | 2.5 | | 3 |
| | cyclodextrin | | 1.65 | |
| | locust bean gum | | | |
| | carboxymethyl cellulose | | | |
| Polyhydric alcohol | glycerin | 20 | 30 | 30 |
| | propylene glycol | | | |
| | D-sorbitol solution (70%) | 2 | 3.75 | 3 |
| | erythritol | | | |
| Crosslinking agent | dried aluminum hydroxide gel | 0.37 | 0.37 | 0.37 |
| | ethylene glycol diglycidyl ether | | 0.1 | 0.1 |
| | urea | | | |
| | synthetic aluminum silicate | | | |
| | magnesium aluminate metasilicate | | | |
| | sodium tetraborate | 0.5 | | |
| pH adjuster | triethanolamine | | | |
| Surfactant | polyethylene glycol monostearate | | 0.3 | 0.3 |
| Preservative | methyl paraben | 0.18 | 0.18 | 0.18 |
| | propyl paraben | 0.02 | 0.02 | 0.02 |
| | purified water | 55.93 | 51.63 | 54.03 |
| | A+B+C | 18 | 10.65 | 9 |
| | A/(A+B+C) | 2.78 | 18.78 | 27.78 |
| | (B+C)/A | 35.00 | 4.33 | 2.60 |
| Gel properties | pH | 6.30 | 6.60 | 6.25 |
| | Probe track (gF) | 14.3±0.5 | 9.8±1.0 | 11.8±1.3 |
| | viscosity (cps) | 22500 | 24000 | 38000 |
| Sensory evaluation | skin adhesion | ○ | ○ | ⓞ |
| | stickiness | ○ | ⓞ | ○ |
| | shape retention | ⓞ | ⓞ | ⓞ |
| | oozing | ⓞ | ⓞ | ⓞ |
| | cup releasability | ⓞ | ⓞ | ○ |
| | overall evaluation (gel properties) | ⓞ | ⓞ | ○ |

**(Table 9)**

| (Comparative Examples 1 to 6) | | | | | | | |
|---|---|---|---|---|---|---|---|
| | Ingredients | 1 | 2 | 3 | 4 | 5 | 6 |
| Drug | dexamethasone sodium phosphate | 3 | 3 | 3 | 3 | 3 | 3 |
| Ionic synthetic polymer (A) | polysodium acrylate | 2 | 3 | 2 | 2.5 | 2.5 | |
| | partially neutralized polyacrylic acid | 0.5 | | 1 | 1 | 1.5 | 4.5 |
| | N-vinylacetoamid e-acrylic acid copolymer | | | | | | |
| Nonionic synthetic polymer (B) | polyvinyl alcohol (partially saponified product) | 1 | 0.5 | 1.5 | 3 | 3 | 3 |
| | polyvinyl alcohol (fully saponified product) | | | | | | |
| | polyvinyl pyrrolidone | | | | | | |
| | polyethylene oxide | | | | | | |
| Naturally-oc curring polymer (C) | gelatin | 2.5 | 3 | 1.5 | 3 | 3 | 3 |
| | carrageenan | | | | | | |
| | cyclodextrin | | | | | | |
| Polyhydric alcohol | glycerin | 30 | 30 | 30 | 30 | 30 | 30 |
| | D-sorbitol solution (70%) | | | 3 | 2 | 2 | 2 |
| Crosslinking agent | dried aluminum hydroxide gel | 0.37 | 0.37 | 0.37 | 0.37 | 0.37 | 0.37 |
| | ethylene glycol diglycidyl ether | 0.1 | 0.1 | 0.1 | 0.1 | 0.1 | 0.1 |
| | urea | | | | | | |
| | magnesium aluminum hydroxide | | | | | | |
| pH adjuster | triethanolamine | 0.5 | | 0.5 | 1 | 1.5 | 1.5 |
| | tartaric acid | | | | | | |
| Surfactant | polyethylene glycol monostearate | 0.5 | 0.3 | 0.3 | 0.5 | 0.5 | 0.5 |
| Preservative | methyl paraben | 0.18 | 0.18 | 0.18 | 0.18 | 0.18 | 0.18 |
| | propyl paraben | 0.02 | 0.02 | 0.02 | 0.05 | 0.05 | 0.05 |
| | purified water | 59.33 | 59.53 | 56.53 | 53.3 | 52.3 | 51.8 |
| | A+B+C | 6 | 6.5 | 6 | 9.5 | 10 | 10.5 |
| | A/(A+B+C) | 41.67 | 46.15 | 50.00 | 36.84 | 40.00 | 42.86 |
| | (B+C)/A | 1.40 | 1.17 | 1.00 | 1.71 | 1.50 | 1.33 |
| Gelled material | pH | 7.67 | 7.89 | 7.78 | 7.29 | 7.75 | 7.52 |
| | Probe track (gF) | 5.0±0.8 | 10.0±1.0 | 8.3±1.3 | 15.0±0.3 | 1.0±0.6 | 12.0±0.6 |
| | viscosity (cps) | 19500 | 24000 | 22500 | 37000 | 40500 | 37500 |
| Sensory evaluation | skin adhesion | Δ | ○ | Δ | ○ | ⓞ | ○ |
| | stickiness | × | × | × | ⓞ | ⓞ | ⓞ |
| | shape retention | ○ | ○ | Δ | ⓞ | ⓞ | ⓞ |
| | oozing | × | × | × | ⓞ | ⓞ | ⓞ |
| | cup releasability | ⓞ | ⓞ | ○ | ⓞ | ⓞ | ⓞ |
| | overall evaluation (gel properties) | × | × | × | ⓞ | ⓞ | ⓞ |

**(Table 10)**

| (Comparative Examples 7 to 11) | | | | | | |
|---|---|---|---|---|---|---|
| | Ingredients | 7 | 8 | 9 | 10 | 11 |
| Drug | dexamethasone sodium phosphate | 3 | 3 | 3 | 3 | 3 |
| Ionic synthetic polymer (A) | polysodium acrylate | | | | | 2 |
| | partially neutralized polyacrylic acid | | | | | |
| | N-vinylacetoamid e-acrylic acid copolymer | | | | 10 | |
| Nonionic synthetic polymer (B) | polyvinyl alcohol (partially saponified product) | | | | | 2 |
| | polyvinyl alcohol (fully saponified product) | 12 | | | | |
| | polyvinyl pyrrolidone | | | | 3 | |
| | polyethylene oxide | | | | | 2 |
| Naturally-oc curring polymer (C) | gelatin | | 4 | | | 3 |
| | carrageenan | | | 3 | | |
| | cyclodextrin | | | | | 3.3 |
| Polyhydric alcohol | glycerin | | 20 | 15 | 40 | 30 |
| | D-sorbitol solution (70%) | | | | | 5 |
| Crosslinking agent | dried aluminum hydroxide gel | | | | | 0.37 |
| | ethylene glycol diglycidyl ether | | | | | 0.1 |
| | urea | | | | 1 | |
| | magnesium aluminum hydroxide | | | | 0.8 | |
| pH adjuster | triethanolamine | | | | | |
| | tartaric acid | | | | 0.6 | |
| Surfactant | polyethylene glycol monostearate | | | | | 0.3 |
| Preservative | methyl paraben | | 0.18 | 0.18 | | 0.18 |
| | propyl paraben | | 0.02 | 0.02 | | 0.02 |
| | purified water | 85 | 72.8 | 78.8 | 41.6 | 48.73 |
| | A+B+C | 12 | 4 | 3 | 13 | 12.3 |
| | A/(A+B+C) | 0.00 | 0.00 | 0.00 | 76.92 | 16.26 |
| | (B+C)/A | - | - | - | 0.30 | 5.15 |
| Gelled material | pH | 7.56 | 7.75 | 7.65 | 7.74 | 7.79 |
| | Probe track (gF) | 0.2±0.1 | 1.2±0.1 | 0.5±0.1 | 14.1±0.1 | 8.8±0.5 |
| | viscosity (cps) | 10000 | 20000 | 5000 | 110000 | 22500 |
| Sensory evaluation | skin adhesion | × | × | × | ○ | ○ |
| | stickiness | ⓞ | Δ | × | Δ | ⓞ |
| | shape retention | ⓞ | ⓞ | Δ | ○ | ⓞ |
| | oozing | Δ | Δ | × | ⓞ | ⓞ |
| | cup releasability | ⓞ | ⓞ | ⓞ | ⓞ | ⓞ |
| | overall evaluation (gel properties) | × | × | × | ○ | ⓞ |

### (Test example 2)

The effect of the constitution ratio of the ionic synthetic polymer, nonionic synthetic polymer and naturally-occurring polymer on the overall evaluation of gel properties, gel viscosity and fillability was examined for each of the compositions of examples 1 to 6, 8 to 11, 13 to 15, 20 to 23 and of comparative examples 1 to 3 and 7 to 10. The results are shown in Table 11.

**(Table 11)**

| Examples | Ionic synthetic polymer (A) | Nonionic synthetic polymer (B) | Naturally-occurring polymer (C) | Constitution ratio (B+C)/A | A+B+C | Overall evaluation of gel properties | Viscosity (cps) | Fillability |
|---|---|---|---|---|---|---|---|---|
| Example 1 | 0.25 | 15 | 2.5 | 70 | 17.75 | ○ | 15500 | ⓞ |
| Example 2 | 0.5 | 15 | 2.5 | 35 | 18.0 | ○ | 28000 | ⓞ |
| Example 3 | 1.0 | 15 | 2.5 | 17.5 | 18.5 | ⓞ | 32000 | ⓞ |
| Example 4 | 1.5 | 4 | 6.3 | 6.87 | 11.8 | ⓞ | 22500 | ⓞ |
| Example 5 | 2 | 4 | 6.3 | 5.15 | 12.3 | ⓞ | 24000 | ⓞ |
| Example 6 | 2 | 3.5 | 5.15 | 4.33 | 10.65 | ⓞ | 24500 | ⓞ |
| Example 8 | 2 | 4 | 3 | 3.50 | 9 | ○ | 23000 | ⓞ |
| Example 9 | 2 | 3 | 3 | 3 | 8 | ○ | 22500 | ⓞ |
| Example 10 | 2 | 2 | 3 | 2.5 | 7 | ○ | 20000 | ⓞ |
| Example 11 | 2 | 1 | 3 | 2 | 6 | ○ | 15000 | ⓞ |
| Example 13 | 2.5 | 3.5 | 5.15 | 3.46 | 11.15 | ⓞ | 27000 | ○ |
| Example 14 | 2.5 | 3.5 | 3 | 2.60 | 9 | ⓞ | 28500 | ⓞ |
| Example 15 | 2.5 | 2 | 3 | 2 | 7.5 | ⓞ | 23000 | ⓞ |
| Example 20 | 3 | 3 | 3 | 2 | 9 | ⓞ | 28500 | ○ |
| Example 21 | 3 | 2.5 | 2.5 | 1.67 | 8 | ⓞ | 27000 | ○ |
| Example 22 | 3 | 2 | 2.5 | 1.5 | 7.5 | ○ | 26500 | ⓞ |
| Example 23 | 3 | 1.5 | 2.5 | 1.33 | 7 | ○ | 25000 | ⓞ |
| Comparative example 1 | 2.5 | 1 | 2.5 | 1.4 | 6 | × | 19500 | ⓞ |
| Comparative example 2 | 3 | 0.5 | 3 | 1.17 | 6 | × | 24000 | ⓞ |
| Comparative example 3 | 3 | 1.5 | 1.5 | 1 | 6 | × | 28500 | ⓞ |
| Comparative example 7 | 0 | 12 | 0 | | 12 | × | 10000 | ⓞ |
| Comparative example 8 | 0 | 0 | 4 | | 4 | × | 2000 | ⓞ |
| Comparative example 9 | 0 | 0 | 3 | | 3 | × | 5000 | ⓞ |
| Comparative example 10 | 10 | 3 | 3 | 1.33 | 16 | ○ | 110000 | × |

The results shown in Table 11 reveal that in the composition of each example of the present invention in which the amount of ionic synthetic polymer (A) mixed is kept within 3% of the entire gel composition, taking into consideration drug absorption of the gel composition, good gel properties are obtained when the composition of the mixture of the ionic synthetic polymer (A), the nonionic synthetic polymer (B) and the naturally-occurring polymer (C) is such that (B + C)/A ≥ 1. 5 and/or A + B + C ≥ 7%. Specifically, the gel viscosity right after the gel preparation was less than 30000 cps and handleability during the gel production was good, and thus the gel compositions were the optimum ones as filling type of gel compositions.

On the other hand, in the gel compositions of comparative examples 1 to 3, in which the composition of each mixture of the ionic synthetic polymer (A), the nonionic synthetic polymer (B) and the naturally-occurring polymer (C) was such that (B + C)/A was less than 1.5 or A + B + C was less than 7%, the gel shape retention was not satisfactory and stickiness and oozing of the gel were significantly observed.

In the gel compositions of comparative examples 7 to 9, in which either one of the nonionic synthetic polymer and the naturally-occurring polymer alone was mixed, their adhesion was not satisfactory and oozing occurred. In the gel composition blended with N-vinylacetoamide of comparative example 10, the gel viscosity was markedly high and the composition showed thixotropic characteristics peculiar to it, though the gel properties were good, and thus its quantitative filling was impossible.

The results so far proved that the adhesive gel composition of this invention is highly useful in terms of functionality and productivity.

### (Experimental example 2: Drug absorption test in rat)

An electrode printed with silver chloride paste was attached to each of the adhesive gel compositions (4.5 cm²) of examples 1 to 6, 8, 9, 13 to 15, 20 23 and 24 and of comparative examples 4 to 10 and used as an donor electrode (cathode). And a formulation (4.5 cm²) prepared by mounting the printed surface of a silver paste-printed electrode with PET nonwoven fabric and impregnated with physiological salt solution was used as a reference electrode (anode).

The formulation was pasted on the shaved abdomen of 7-week-old SD rats (body weight 250 g), fixed with Elatex Tape (ALCARE), and connected with a cord, which is connected to a power source and a recorder, to start energizing (constant current: 0.10 mA/cm², energizing time: 3 hr). Blood was collected transvenously from the rats using a syringe, and the blood plasma was dephosphorylated with alkaline phosphatase, cleaned up with an ODS cartridge, and quantified by HPLC as dexamethasone. The results are shown in Table 12.

**(Table 12)**

| Examples | Ionic synthetic polymer (A) | Nonionic synthetic polymer (B) | Naturally-occ urring polymer (C) | Constitution ratio (A+B)/C | Dexamethasone blood level (3 hr mean ± SD) |
|---|---|---|---|---|---|
| Example 1 | 0.25 | 15 | 2.5 | 70 | 784.17±51.22 |
| Example 2 | 0.5 | 15 | 2.5 | 35 | 791.34±99.43 |
| Example 3 | 1.0 | 15 | 2.5 | 17.5 | 773.39±39.87 |
| Example 4 | 1.5 | 4 | 6.3 | 6.87 | 741.42±73.85 |
| Example 5 | 2 | 4 | 6.3 | 5.15 | 774.75±50.17 |
| Example 6 | 2 | 3.5 | 5.15 | 4.33 | 797.25±113.62 |
| Example 8 | 2 | 4 | 3 | 3.50 | 796.50±104.18 |
| Example 9 | 2 | 3 | 3 | 3 | 867.25±52.13 |
| Example 13 | 2.5 | 3.5 | 5.15 | 3.46 | 681.71±66.19 |
| Example 14 | 2.5 | 3.5 | 3 | 2.60 | 659.68±44.51 |
| Example 15 | 2.5 | 2 | 3 | 2 | 701.38±56.20 |
| Example 20 | 3 | 3 | 3 | 2 | 567.27±26.90 |
| Example 23 | 3 | 2 | 2.5 | 1.5 | 556.96±45.82 |
| Example 24 | 3 | 3 | 3 | 2 | 520.21±72.65 |
| Comparative example 4 | 3.5 | 3 | 3 | 1.71 | 276.00±52.18 |
| Comparative example 5 | 4.0 | 3 | 3 | 1.50 | 156.90±29.02 |
| Comparative example 6 | 4.5 | 3 | 3 | 1.33 | 114.46±20.30 |
| Comparative example 7 | 0 | 12 | 0 | - | 808.88±123.40 |
| Comparative example 8 | 0 | 0 | 4 | - | 765.64±75.32 |
| Comparative example 9 | 0 | 0 | 3 | - | 785.36±740.33 |
| Comparative example 10 | 10 | 3 | 3 | 1.33 | 726.71±46.03 |

The results shown in Table 12 reveal that the blood level of the drug was decreased with the increase in the amount of the ionic synthetic polymer mixed, irrespective of the amount of the nonionic synthetic polymer and the naturally-occurring polymer mixed, and the decrease in the blood level of the drug was noticeable particularly when the amount of the ionic synthetic polymer mixed was 3.5% or more (comparative examples 4 to 6). The reasons for this are probably that the ionic synthetic polymer has dissociable functional groups, and therefore it is electrically conductive and it decreases the amount of free water in the gel.

The formulation of comparative example 10 uses weakly ionic synthetic polymer, N-vinylacetoamide. The experimental result show that with such a base, the blood level of the drug did not decrease very much. However, as shown in Table 8, in the gel composition of comparative example 10, its viscosity was significantly high and its fillability was very poor.

The results shown so far indicate that in iontophoretic drug delivery, if the iontophoretic formulation contains 3% by weight or more of ionic synthetic polymer that has dissociable functional groups, negative effect is produced in terms of drug absorption.

### (Test example 3: Drug efficacy test)

The inflammation inhibitory effect in rabbit chronic arthritis models was examined using the formulations of examples 2, 8, 15 and 20 and of comparative examples 5, 6 and 10. The methods for preparing the models and evaluating the effect were as follows. <Preparation of BSA induced arthritis models> After dosing with methylated BSA, booster was carried out in rabbits three times to raise BSA antibody in them. After confirming the antibody was raised, 0.5 ml of 0.1% m BSA was injected into the joint of each rabbit's right ankle to induce inflammation. Inflammation was induced every other week four times. Twenty-four hours after the fourth induction of inflammation, the degree of the inflammation was measured to group the rabbits by the degree, and drug administration was performed. Each rabbit was given single intra-articularly injections of 0.5 ml of physiological salt solution in its left ankle, as a contrast. <Method for evaluation> The width (lateral length) of each joint was measured and the degree of inflammation was evaluated at 24^{th} hr, 48^{th} hr, 72^{nd} hr and 96^{th} hr. The results are shown in Figure 1.

The results shown in Figure 1 reveal that the smaller the amount of the ionic synthetic polymer mixed became, the higher the degree of inhibiting the inflammation became, irrespective of the amount of the nonionic synthetic polymer and the naturally-occurring polymer mixed. This is correlated with the blood level of the drug shown in test example 2 (Table 12), and it has been proved that it is preferable in terms of drug efficacy to decrease the amount of the ionic synthetic polymer mixed.

### (Test example 4: drug stability test)

Formulations prepared by attaching an electrode printed with silver chloride paste to each of the adhesive gel compositions (4.5 cm²) of examples 2, 4 to 6, 8, 13, 15, 20 and 22 and of comparative examples 7 to 11 were wrapped in aluminum foil and stored at 50°C and 75% RH to examine the degree of change in active ingredient (dexamethasone sodium phosphate) with time. The fluctuation in pH by drug decomposition was also examined by measuring the pH of the gel surface. The results are shown in Table 13. The rate of the drug remaining in the gel is shown by mean ± standard deviation (N = 3).

**(Table 13)**

| Examples | Ionic synthetic polymer (A) | pH (initial) | pH (50°C - one month) | pH fluctuation | Rate of remaining drug 50°C - one month (% to initial value) |
|---|---|---|---|---|---|
| Example 2 | 0.5 | 6.76 | 6.31 | 0.45 | 86.58±1.66 |
| Example 4 | 1.5 | 6.81 | 6.69 | 0.12 | 92.28±1.17 |
| Example 5 | 2 | 7.86 | 7.80 | 0.06 | 94.63±0.62 |
| Example 6 | 2 | 7.85 | 7.81 | 0.04 | 95.72±0.76 |
| Example 8 | 2 | 7.46 | 7.42 | 0.04 | 94.65±0.44 |
| Example 13 | 2.5 | 7.72 | 7.64 | 0.08 | 92.65±0.86 |
| Example 15 | 2.5 | 6.89 | 6.83 | 0.06 | 94.49±0.63 |
| Example 20 | 3 | 7.83 | 7.81 | 0.02 | 98.65±0.77 |
| Example 22 | 3 | 7.79 | 7.78 | 0.01 | 98.78±1.17 |
| Comparative example 7 | 0 | 7.76 | 7.26 | 0.50 | 85.24±0.62 |
| Comparative example 8 | 0 | 7.50 | 6.85 | 0.65 | 82.14±2.20 |
| Comparative example 10 | 10 | 7.53 | 7.13 | 0.40 | 86.70±1.21 |
| Comparative example 11 | 2 | 7.85 | 7.67 | 0.18 | 90.50±0.68 |

The results shown in Table 13 reveal that the larger the amount of the ionic synthetic polymer mixed became, the more the drug stability with time was improved. They also reveal that the fluctuation in pH of gel with time was smaller in compositions whose stability with time was high.

The results shown so far prove that ionic synthetic polymer forms a high pH buffer capacity, thereby being capable of suppressing pH fluctuation by hydrolysis etc. and decreasing the decomposition speed of drugs. The present invention has been made based on this finding.

Further, the results of example 15 and comparative example 11 shown in Table 13 reveal that according to the production method of the present invention, the ingredients of the gel composition can be compounded by nitrogen treatment or under vacuum, whereby the amount of oxygen dissolved in the gel can be greatly decreased, and hence the speed of drugs' decomposition by oxidation.

These results indicate that according to the method for producing an adhesive gel composition of the present invention, even drugs whose stability is very hard to keep from hydrolysis and oxidative decomposition can be mixed into a gel composition, since the method enables the suppression of both hydrolysis and oxidative decomposition of drugs.

### Industrial Applicability

The adhesive gel composition of the present invention does not show thixotropic characteristics, which are peculiar to gel properties during and after the gel preparation, and it can be continuously and easily prepared and filled because its viscosity can be set to a mid degree or smaller degree, and therefore, it can be produced at low costs. Furthermore, since the gel can be produced by filling of nitrogen or under vacuum, even drugs less stable to hydrolysis or oxidation can be compounded in a stable state.

The adhesive gel composition of the present invention does not cause deterioration in drug absorption due to the decrease in transport number of drugs and the decrease in free water, which is a disadvantage of conventional gel compositions that contain an ionic synthetic polymer(s), has good drug releasability, and excels in both shape retention and adhesion.

The adhesive gel composition and the method for producing the same in accordance with this invention show excellent characteristics in handleability, productivity, stability with time, drug releasability, drug efficacy and economy, and hence are very suitably applied to iontophoretic formulations.

## Claims

1. An adhesive gel composition for iontophoretic formulations, comprising an ionic synthetic polymer(s), a nonionic synthetic polymer(s), a naturally-occurring polymer(s), a polyhydric alcohol(s), a crosslinking agent(s) and a drug(s).

2. The adhesive gel composition for iontophoretic formulations according to claim 1, wherein the amount of the ionic synthetic polymer(s) mixed is 0.1 to 3.0% by weight, the amount of the nonionic synthetic polymer(s) mixed is 0.5 to 30.0% by weight, the amount of the naturally-occurring polymer(s) mixed is 0.5 to 10.0% by weight and the amount of the polyhydric alcohol (s) mixed is 1.0 to 60.0% by weight.

3. The adhesive gel composition for iontophoretic formulations according to claim 1 or 2, wherein the composition (weight) of the mixture of the ionic synthetic polymer(s) (A), the nonionic synthetic polymer(s) (B) and the naturally-occurring polymer(s) (C) is such that
(B + C)/A ≥ 1.5 % by weight and/or A + B + C ≥ 7% by weight.

4. The adhesive gel composition for iontophoretic formulations according to any of claims 1 to 3, wherein the ionic synthetic polymer (s) is or are obtained by polymerizing polymerizable unsaturated monomers that have at least anionic functional groups.

5. The adhesive gel composition for iontophoretic formulations according to any of claims 1 to 4, wherein the ionic synthetic polymer(s) comprises one or more substances selected from the group consisting of polyacrylic acid, partially neutralized polyacrylic acid, fully neutralized polyacrylic acid, methoxyethylene-maleic anhydride copolymer, methoxyethylene-maleic acid copolymer, isobutylene-maleic anhydride copolymer, isobutylene-maleic acid copolymer and carboxyvinyl polymer.

6. The adhesive gel composition for iontophoretic formulations according to any of claims 1 to 5, wherein the nonionic synthetic polymer(s) comprises one or more substances selected from the group consisting of polyvinyl alcohol, polyvinyl pyrrolidone and polyethylene oxide.

7. The adhesive gel composition for iontophoretic formulations according to any of claims 1 to 6, wherein the naturally-occurring polymer(s) comprises one or more substances selected from the group consisting of gelatin, carrageenan, locust bean gum, dextrin, carboxymethyl cellulose and the metallic salt of carboxymethyl cellulose.

8. The adhesive gel composition for iontophoretic formulations according to any of claims 1 to 7, wherein the polyhydric alcohol(s) is one, or are two or more selected from the group consisting of glycerin, polyethylene glycol, propylene glycol, D-sorbitol, xylitol, mannitol and erythritol.

9. The adhesive gel composition for iontophoretic formulations according to any of claims 1 to 8, wherein the crosslinking agent(s) is one, or are two or more selected from the group consisting of polyvalent metallic compounds, polyfunctional epoxy compounds and boric acid-based compounds.

10. The adhesive gel composition for iontophoretic formulations according to any of claims 1 to 9, wherein the drug forms anions in the adhesive gel composition for iontophoretic formulations and can be delivered from the cathode side of the iontophoretic formulations.

11. The adhesive gel composition for iontophoretic formulations according to claim 10, wherein the drug(s) is(are) a water-soluble steroid hormone(s).

12. The adhesive gel composition for iontophoretic formulations according to claim 11, wherein the water-soluble steroid hormone (s) is one, or are two or more selected from the group consisting of dexamethasone sodium phosphate, dexamethasone sodium acetate, dexamethasone sodium metasulfonbenzoate, hydrocortisone sodium succinate, hydrocortisone sodium phosphate, prednisolone sodium succinate and betamethasone sodium phosphate.

13. The adhesive gel composition for iontophoretic formulations according to any of claims 1 to 12, wherein the composition has pH ranging from 4 to 9.

14. The adhesive gel composition for iontophoretic formulations according to any of claims 1 to 13, wherein oxygen dissolved in the gel is positively removed by replacement with nitrogen and/or vacuum kneading at the time the ingredients are added and kneaded.

15. A method for producing an adhesive gel composition for iontophoretic formulations, wherein oxygen dissolved in the gel is positively removed by carrying out replacement with nitrogen and/or vacuum kneading at the time ingredients, including an ionic synthetic polymer(s), a nonionic synthetic polymer(s), a naturally-occurring polymer(s), a polyhydric alcohol(s), a crosslinking agent(s) and a drug(s), are mixed and kneaded.
